# FASCICULE DE BREVET EUROPEEN

(11) **EP 0 243 284 B1**
(45) Date de publication et mention de la délivrance du brevet: **17.02.1993**
(21) Numéro de dépôt: 87420100.7
(22) Date de dépôt: 14.04.1987
(51) Int. Cl.: A61M 1/16

(54) **Rein artificiel avec dispositif de contrôle des quantités de liquide circulant dans le circuit de liquide de dialyse**
Künstliche Niere mit einer Anordnung zur Kontrolle der im Dialysatkreislauf fliessenden Flüssigkeitsmengen
Artificial kidney with a device for controlling the volumes of fluids flowing trough the dialysate circuit

(30) Priorité: 25.04.1986 FR 8606225
(43) Date de publication de la demande: 28.10.1987
(73) Titulaire: HOSPAL INDUSTRIE, F-69330 Meyzieu (FR)
(72) Inventeur: Chevallet, Jacques, F-69360 Serezin du Rhône (FR)

(56) Documents cités:
- EP-A- 0 122 604
- DE-A- 3 439 661
- US-A- 3 946 731
- THE INTERNATIONAL JOURNAL OF ARTIFICIAL ORGANS, vol. 8, no. 1, 1985, pages 33-36; J.M.P. WOKKE et al.: "Volumetrical microcomputer-based ultrafiltration monitor for hemodialysis"

## Description

La présente invention concerne un rein artificiel utilisable dans le traitement extracorporel du sang, notamment par dialyse et par ultrafiltration. Plus particulièrement, la présente invention concerne un rein artificiel permettant de contrôler avec une grande précision l'ultrafiltration du sang lors d'une séance d'hémodialyse.

La présente invention concerne également un procédé de contrôle et de maintien, pour un rein artificiel, de l'égalité des quantités de liquide de dialyse entrant et sortant dans le circuit de liquide de dialyse.

Des reins artificiels, mettant en oeuvre le contrôle de l'ultrafiltration sont déjà connus dans l'état de la techinique. Ainsi, par l'article "Volumetrical microcomputer based ultrafiltration monitor for hemodialysis" de J.M.P. Wokke (the International Journal of Artificial Organs (Vol. 8 No 1, 1985), on connait un rein artificiel dont le circuit de liquide de dialyse comporte deux débitmètres l'un en amont, l'autre en aval de l'hémodialyseur. Par suite du gradient de pression exercé de part et d'autre de la membrane, une fraction du liquide présent dans le sang ultrafiltre à travers la membrane. Le débit du liquide de dialyse mesuré en aval de l'hémodialyseur est alors supérieur au débit mesuré en amont de l'hémodialyseur. La différence entre la mesure du débit aval et celle du débit amont est considérée correspondre au débit d'ultrafiltration. Dans un tel système, les erreurs intrinsèques aux débitmètres sont en partie corrigées par un étalonnage réalisé en court-circuitant l'hémodialyseur (pas d'ultrafiltration) et en faisant circuler le même débit de liquide de dialyse dans les deux débitmètres. Or, lorsque l'appareil est en fonctionnement normal, c'est à dire lorsqu'il y a ultrafiltration, le débit mesuré en aval de l'hémodialyseur n'est plus le seul débit de liquide de dialyse mais est la somme du débit de liquide de dialyse et du débit d'ultrafiltrat. La mesure du débit aval manque alors de précision car la correction apportée par l'étalonnage n'est valable que pour une valeur de débit égale à celle de l'étalonnage, ce qui entraine également un manque de précision sur le contrôle de l'ultrafiltration.

On connait, d'autre part, par le brevet US 3.946.731 de Lichtenstein, un rein artificiel comportant en série sur le liquide de dialyse (cf. figure II) : un réservoir de liquide de dialyse, une pompe amont, un débitmètre amont, un hémodialyseur, un débitmètre aval, une pompe aval. Un dispositif pour recueillir l'ultrafiltrat est placé en dérivation entre le débitmètre et la pompe aval, ce dispositif est destiné à recevoir l'excès de liquide de dialyse correspondant à l'ultrafiltrat provenant du sang. Dans un tel appareil, les pompes aval et amont sont mises en oeuvre de façon synchrone, de telle manière qu'elles débitent les mêmes quantités de liquide de dialyse à l'entrée et à la sortie du circuit de liquide de dialyse, à la précision des pompes près. Cet appareil et cette méthode pour contrôler l'ultrafiltration manquent de précision car les erreurs sur la mesure de l'ultrafiltration résultent notamment des erreurs provenant de chacun des débitmètres amont et aval ainsi que des erreurs sur l'égalité des débits délivrés par les pompes disposées en amont et en aval de l'hémodialyseur.

La mesure de l'ultrafiltration selon l'enseignement de ce brevet ne permet pas une précision suffisante sur le contrôle de l'ultrafiltration. En effet, si l'on veut satisfaire aux exigences actuelles du marché, il faut pouvoir mesurer les quantités de liquide de dialyse à l'entrée et à la sortie du circuit de liquide de dialyse à plus ou moins 30 ml/heure.

L'objet de la présente invention est donc de proposer un rein artificiel qui ne présente pas les inconvénients de l'art antérieur et qui permet de contrôler, de façon très précise, l'ultrafiltration du sang lors d'une séance d'hémodialyse.

Un autre objet de la présente invention est de proposer un rein artificiel comportant une portion de circuit de liquide de dialyse pour laquelle, qu'il y ait ou non ultrafiltration, la quantité de liquide de dialyse qui entre est maintenue égale à la quantité de liquide de dialyse qui sort, ceci avec une très grande précision.

C'est encore un objet de la présente invention que de proposer un rein artificiel permettant de s'affranchir des erreurs intrinsèques aux capteurs utilisés pour mesurer les quantités de liquide de dialyse circulant.

D'autres objets et avantages relatifs à la présente invention apparaîtront à la lecture de la description qui va suivre.

Pour faciliter la compréhension de l'invention, on désignera par capteur amont le capteur disposé en amont de l'hémodialyseur et par capteur aval le capteur disposé en aval de l'hémodialyseur.

Il a maintenant été trouvé, et c'est ce qui fait l'objet de la présente invention, un rein artificiel permettant d'assurer la mesure et le contrôle, avec une très grande précision, des quantités de liquide de dialyse circulant en amont et en aval de l'hémodialyseur.

La présente invention concerne donc un rein artificiel comportant un hémodialyseur comprenant au moins deux compartiments séparés par une membrane semi-perméable permettant la dialyse et l'ultrafiltration du sang, le premier compartiment étant destiné à être relié à un patient par un circuit extracorporel de sang, le second compartiment étant traversé par un circuit de liquide de dialyse, ledit circuit de liquide de dialyse comportant en amont et en aval de l'hémodialyseur un moyen pour déplacer le liquide de dialyse ainsi qu'un capteur apte à mesurer la quantité de liquide de dialyse circulant et comportant en outre des moyens pour comparer les valeurs obtenues par le capteur amont et les valeurs obtenues par-le capteur aval pendant la même période .

Ce rein artificiel est caractérisé en ce qu'il comporte des moyens pour maintenir l'égalité entre les valeurs obtenues par le capteur amont et les valeurs obtenues par le capteur aval pendant la même période, lesdits moyens comportant des moyens pour asservir le fonctionnement de l'un au, moins des moyens pour déplacer le liquide de dialyse au produit de la comparaison entre les valeurs obtenues par le capteur amont et les valeurs obtenues par le capteur aval pendant la même période. La présente invention concerne donc un rein artificiel comportant des moyens pour que les quantités de liquide de dialyse circulant dans le capteur amont soient maintenues rigoureusement égales aux quantités de liquide de dialyse circulant dans le capteur aval, durant l'ensemble de la séance de dialyse.

Le rein artificiel selon la présente invention comporte en outre des moyens pour retirer du liquide de dialyse hors de la portion du circuit de liquide de dialyse traversant l'hémodialyseur et comprise entre les capteurs amont (6) et aval (7).
Ces moyens pour retirer du liquide de dialyse hors du circuit (5) peuvent comporter des moyens (20) pour laisser s'écouler le liquide de dialyse, ou encore, de façon préférentielle, des moyens (17) pour imposer, grâce à une pompe occlusive par exemple, l'extraction d'une quantité prédéterminée de liquide de dialyse.

Dans le cas où on impose, par une pompe par exemple, l'extraction du liquide de dialyse, l'action combinée de cette pompe occlusive (17) et des moyens pour déplacer le liquide de dialyse (9,10) entraîne une dépression dans la portion du circuit de liquide de dialyse traversant l'hémodialyseur. Cette dépression entraîne, si le circuit est indéformable, le passage à travers la membrane semi-perméable de l'hémodialyseur d'une fraction du liquide présent dans le sang, jusqu'à rétablir l'équilibre entre les pressions du sang et du liquide de dialyse. La quantité d'ultrafiltrat ainsi obtenue est alors égale à la quantité de liquide de dialyse extraite du circuit.

Le rein artificiel, objet de la présente invention comporte également des moyens pour effectuer un étalonnage des capteurs. L'étalonnage est réalisé en court-circuitant l'hémodialyseur et le circuit d'ultrafiltration grâce à un circuit de dérivation.

L'objet de la présente invention consiste également en un procédé de contrôle et de maintien de l'égalité des quantités de liquide de dialyse à l'entrée et à la sortie d'un circuit de liquide de dialyse relié à un hémodialyseur et comportant de part et d'autre dudit hémodialyseur au moins un capteur (6,7) apte à mesurer la quantité de liquide de dialyse circulant ainsi qu'un moyen (9,10) pour déplacer le liquide de dialyse, le procédé consistant à :
- mesurer la quantité de liquide de dialyse circulant dans le capteur en amont de l'hémodialyseur,
- mesurer la quantité de liquide de dialyse circulant dans le capteur en aval de l'hémodialyseur pendant la même periode,
- comparer les quantités de liquide de dialyse circulant dans le capteur amont et les quantités de liquide de dialyse circulant dans le capteur aval pendant la même période, et
- asservir le fonctionnement de l'un au moins des moyens pour déplacer le liquide de dialyse au produit de la comparaison entre les quantités de liquide de dialyse circulant dans le capteur amont et les quantités de liquide de dialyse circulant dans le capteur aval pendant la même période .

La compréhension de la présente invention sera facilitée par la description faite ci-dessous en référence aux dessins qui illustrent, à titre d'exemples, schématiquement et sans échelle déterminée, divers modes de réalisation de l'objet de l'invention.

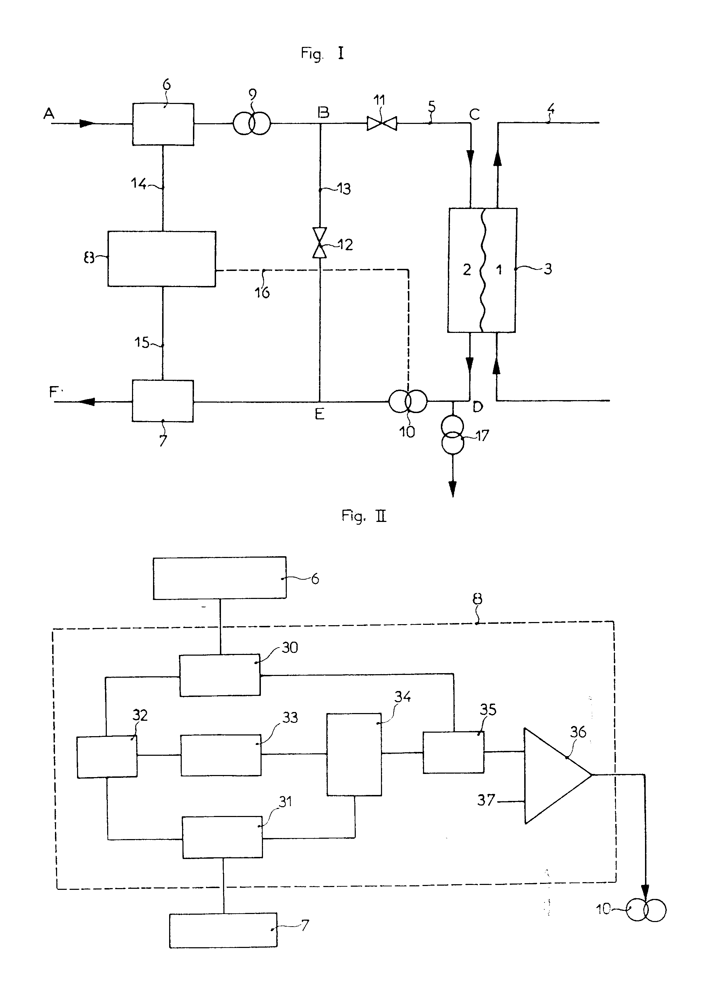

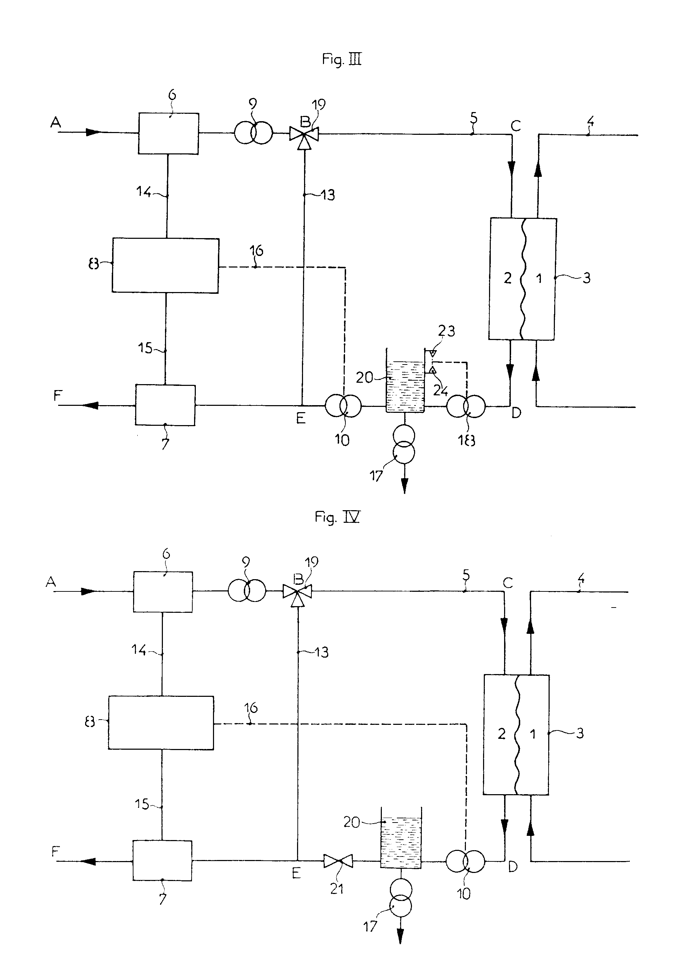

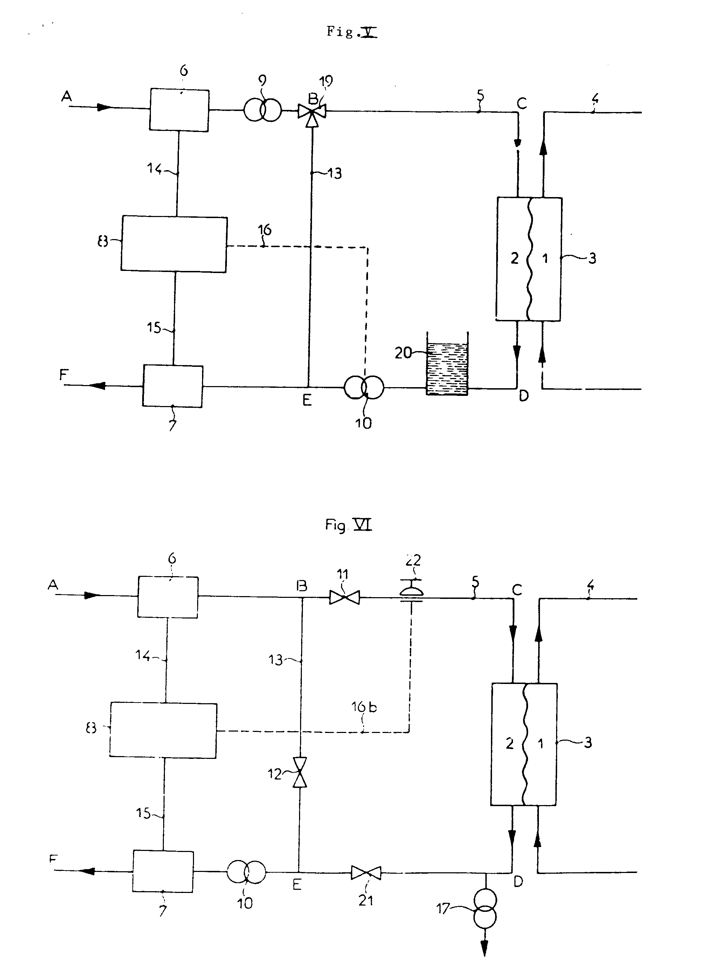

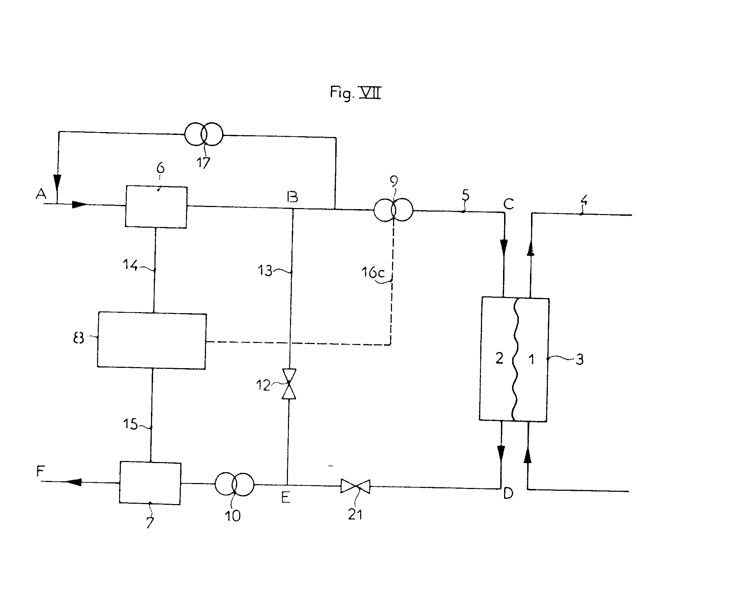

La figure I est un schéma d'un premier mode de réalisation du rein artificiel selon l'invention.

La figure II est un schéma par blocs d'un dispositif de contrôle selon un mode particulier de mise en oeuvre de l'invention.

La figure III est un schéma d'un mode de réalisation préféré du rein artificiel selon l'invention.

Les figures IV à VII sont des schémas de variantes de réalisation du rein artificiel selon l'invention.

En se référant à la figure I, on voit que le rein artificiel selon la présente invention comporte un hémodialyseur (3) de tout type connu dont le premier compartiment (1) est relié à un patient par une circuit extracorporel de sang (4), alors que le second compartiment (2) est traversé par un circuit de liquide de dialyse (5) dont l'entrée (A) est reliée à une source de liquide de dialyse (non représentée) et la sortie (F) à des moyens d'évacuation ou de recyclage (non représentés).

Ledit circuit de liquide de dialyse (5) comporte en amont de l'hémodialyseur (3) un capteur (6) qui mesure la quantité de liquide de dialyse qui circule, en générant par exemple une impulsion au passage d'une fraction prédéterminée de liquide de dialyse. On peut, par exemple, choisir un capteur qui génère une impulsion pour 0,08 ml de liquide de dialyse circulant.

Un exemple de capteur convenant bien pour la présente invention est un débitmètre volumétrique à palettes, modèles T.I.T 115 fabriqué par la société JENCON'S.

Une pompe (9) disposée en aval du capteur (6) déplace le liquide de dialyse. Cette pompe (9) , de préférence occlusive, est une pompe de type connu, par exemple une pompe péristaltique, ou une pompe à engrenages, ou encore une pompe à piston et clapets.

On dispose en aval de la pompe (9) et en amont de l'hémodialyseur (3) une vanne (11) ou tout autre dispositif d'obturation de type connu .

Sur la portion du circuit de liquide de dialyse en aval de l'hémodialyseur, une pompe de circulation (10), par exemple de même type que la pompe (9), déplace le liquide de dialyse.

Un capteur (7) disposé en aval de la pompe (10) mesure la quantité de liquide de dialyse circulant dans cette portion aval du circuit. Avantageusement, ce capteur (7) est de même type que le capteur (6).

On pourrait aussi, bien que cela ne soit pas préférentiel pour la mise en oeuvre de la présente invention, placer l'une des pompes (9,10) ou même les deux, dans la partie externe du circuit de liquide de dialyse, c'est à dire dans la partie du circuit (5) en amont du capteur (6) et en aval du capteur (7).

On dispose un conduit de dérivation (13) entre les portions amont et aval du circuit de liquide de dialyse. Ce conduit de dérivation (13) relie le point (B) situé en aval de la pompe (9) et en amont de la vanne (11) au point (E) situé en aval de la pompe (10) et en amont du capteur (7).

Ce conduit de dérivation (13) permet, avec la fermeture de la vanne (11), lors de l'étalonnage par exemple, d'éviter la circulation du liquide de dialyse dans l'hémodiayseur et le circuit d'ultrafiltration. Ce conduit de dérivation (13) est muni d'une vanne d'arrêt (12) ou de tout autre dispositif d'obturation de type connu.

Un dispositif de contrôle (8) est connecté par une liaison (14) au capteur amont (6) et par une liaison (15) au capteur aval (7).

La régulation de la pompe (10) par le dispositif de contrôle (8) est représentée par la liaison (16) en traits discontinus.

Les fonctions remplies par le dispositif de contrôle (8) seront mieux comprises à l'aide de la figure II qui représente, sous la forme d'un schéma par blocs, un exemple de réalisation dudit dispositif de contrôle (8). Les capteurs (6,7) émettent des impulsions qui sont comptées ou décomptées par les compteurs (30,31). Le correcteur (34) corrige les valeurs de comptage par un facteur correctif déterminé au moment de l'étalonnage par le calculateur (32) et mis en mémoire dans la mémoire (33). On n'effectue, préférentiellement, la correction, que pour une des valeurs de comptage, l'autre valeur de comptage étant directement transmise au comparateur (35). Ledit comparateur (35) compare ensuite le nombre d'impulsions, en valeur corrigée, fournies par chacun des capteurs (6,7) et envoie sur le moniteur (36) un signal d'erreur. Ledit moniteur (36) compare le signal d'erreur reçu à une référence choisie (37) et régule le fonctionnement de la pompe (10) de manière à maintenir le signal d'erreur égal à la référence (37), et par conséquent, les quantités de liquide de dialyse circulant dans le capteur amont (6) égales aux quantités de liquide de dialyse circulant dans le capteur aval (7) pendant la même période.

Dans le cas où la comparaison dans le comparateur (35) entre le nombre d'impulsions émises par le capteur amont (6) et le nombre d'impulsions émises par le capteur aval (7) pendant la même période s'effectue par différence, la référence choisie (37) est 0 (zéro) ; dans le cas où la comparaison s'effectue au moyen d'un rapport, la référence choisie pour le signal d'erreur est alors 1.

Les différents éléments du dispositif de contrôle (8) sont classiques , aussi leur construction spécifique n'est-elle pas considérée comme faisant partie de l'invention.

Préférentiellement, le dispositif de contrôle (8) est un microprocesseur.

Une pompe (17) , de préférence une pompe occlusive, par exemple à engrenages, permet d'extraire hors du circuit de liquide de dialyse des montants prédéterminés (volume et/ou débit) de liquide.

On peut effectuer cette extraction du liquide de dialyse en tout point du circuit (5) situé dans la portion traversant l'hémodiayseur et comprise entre les capteurs amont et aval. En effet, le liquide de dialyse extrait crée dans cette portion de circuit une dépression qui entraîne le passage à travers la membrane semi-perméable de l'hémodialyseur d'une quantité de liquide provenant du sang. La quantité d'ultrafiltrat provenant du sang est égale à la quantité de liquide de dialyse extrait du circuit (5) par la pompe (17), lorsque la partie du circuit en dépression est indéformable.

Le fonctionnement du rein artificiel selon le schéma de la figure I est le suivant . On effectue initialement l'étalonnage des deux capteurs (6,7). La pompe (10) étant arrêtée, on ferme la vanne (11) et on ouvre la vanne (12). L'hémodialyseur et le circuit d'ultrafiltration sont alors court-circuités ; le liquide de dialyse déplacé par la pompe (9) s'écoule dans le circuit de dérivation selon ABEF. Durant cette phase d'étalonnage, la quantité de liquide de dialyse circulant dans le capteur amont (6) est rigoureusement égale à la quantité de liquide de dialyse circulant dans le capteur aval (7) pendant la même période car il ne peut y avoir en aucun point de ce circuit de perte ou de gain de liquide de dialyse.

Cependant, même dans le cas où on utilise en amont et en aval, deux capteurs de même type, de légères différences peuvent exister d'un capteur à l'autre et le signal émis par l'un des capteurs n'est pas toujours rigoureusement identique au signal émis par l'autre capteur pendant la même période. Ainsi, par exemple, dans le cas où on utilise comme capteurs des débitmètres à palettes, le nombre d'impulsions émises par un débitmètre peut différer du nombre d'impulsions émises pendant la même période par l'autre débitmètre de 6 à 7%. L'étalonnage permet de pallier à cet inconvénient ; il permet aussi d'utiliser en amont et en aval deux capteurs de type différent. A cet effet le compteur (30) compte le nombre d'impulsions émises par le capteur amont (6) pendant une période prédéterminée ; le compteur (31) compte, pendant la même période, le nombre d'impulsions émises par le capteur aval (7). Les deux valeurs de comptage sont transmises au calculateur (32) qui détermine le facteur de correction qui peut être, par exemple, le rapport du nombre d'impulsions comptées par l'un des compteurs au nombre d'impulsions comptées par l'autre compteur pendant la même période. Le facteur de correction est mis en mémoire dans la mémoire (33).

La correction, par ce facteur, des mesures effectuées ultérieurement permet de s'affranchir des erreurs dues à la constitution des débitmètres.

Après l'étalonnage, on ouvre la vanne (11) et on ferme la vanne (12). Le liquide de dialyse circule alors dans tout le circuit (5) ABCDEF et notamment dans le compartiment (2) de l'hémodialyseur.

Les quantités de liquide de dialyse débitées par la pompe (9) restant de préférence égales aux quantités débitées pendant l'étalonnage, on règle le fonctionnement de la pompe d'extraction (17) aux valeurs choisies pour les quantités d'ultrafiltrat que l'on veut recueillir.

Les impulsions émises par le capteur amont (6) sont comptées par le compteur (30), celles émises par le capteur aval (7) sont comptées par le compteur (31) pendant la même période. Les valeurs de comptage sont transmises au correcteur qui, avantageusement, ne corrige qu'une valeur de comptage par rapport à l'autre, en fonction du facteur de correction déterminé par le calculateur (32) lors de l'étalonnage et mis en mémoire en (33). La valeur de comptage que l'on corrige peut être celle du compteur (30) ou celle du compteur (31).

Le comparateur (35) compare ensuite le nombre d'impulsions provenant du capteur amont (6) et le nombre d'impulsions provenant du capteur aval (7) pendant la même période . Le comparateur (35) peut, par exemple, effectuer la différence ou le rapport entre le nombre d'impulsions provenant du capteur amont (6) et le nombre d'impulsions provenant du capteur aval (7). Le produit de cette comparaison, ou signal d'erreur, est transmis au moniteur (36) qui compare le signal d'erreur à sa valeur de référence ("0" pour une comparaison par différence, "1" pour une comparaison au moyen d'un rapport).

En fonction de l'écart entre le signal d'erreur et la valeur de référence, le moniteur (36) régule le fonctionnement de la pompe (10), de manière à maintenir le nombre d'impulsions comptées par le compteur (30) égal, après correction, au nombre d'impulsions comptées par le compteur (31) pendant la même période, ce qui conduit à maintenir les quantités de liquide de dialyse circulant dans le capteur amont (6) égales aux quantités de liquide de dialyse circulant dans le capteur aval (7) pendant la même période.

Un mode préfére de réalisation du rein artificiel selon l'invention est celui représenté sur le schéma de la figure III.

Le rein artificiel selon ce mode préféré comporte de la même façon que précédemment sur le circuit de liquide de dialyse : un capteur amont (6), une pompe amont (9), un capteur aval (7), une pompe aval (10), un circuit de dérivation (13), un dispositif de contrôle (8). L'ensemble constitué par les deux vannes (11,12) est remplacé ici par une vanne à trois voies (19) disposée au point B, en aval de la pompe (9) et en amont de l'hémodialyseur (3) à l'intersection avec le circuit de dérivation (13). La mise en oeuvre de cette vanne (19) permet de sélectionner le passage du liquide de dialyse, soit dans le circuit de dérivation (13) soit dans l'hémodialyseur (3).

Selon ce mode de réalisation, le circuit de liquide de dialyse comporte en outre un dispositif permettant d'éliminer les bulles de gaz présentes dans le liquide de dialyse. Ce dispositif de dégazage est constitué, par exemple, par un petit réservoir (20) ouvert à l'atmosphère, en série sur le circuit de liquide de dialyse.

La hauteur du liquide de dialyse dans le réservoir peut varier à l'intérieur de limites déterminées par la position de deux électrodes (23,24). Le niveau du liquide de dialyse est maintenu à l'intérieur de ces limites grâce à la pompe (18) qui est contrôlée par le niveau du liquide de dialyse dans le réservoir. Ainsi, lorsque la hauteur du liquide de dialyse atteint l'électrode de niveau haut (23), celle-ci commande le ralentissement du mouvement de la pompe (18). A l'inverse, lorsque la hauteur de liquide de dialyse atteint l'électrode de niveau bas (24), celle-ci commande l'accélération du mouvement de la pompe (18). La variation possible du volume de liquide de dialyse entre le niveau haut et le niveau bas n'excède en général pas 2 à 3 ml.

L'ouverture du circuit de liquide de dialyse à l'atmosphère permet d'éliminer les bulles de gaz présentes dans le liquide de dialyse. Ainsi, lorsqu'on soutire, par la pompe (17) une quantité de liquide de dialyse qui sera remplacée par une égale quantité d'ultrafiltrat provenant du sang, on a la certitude de ne soutirer que du liquide de dialyse et non pas un mélange de liquide et de bulles de gaz. D'autre part, lorsque les capteurs utilisés pour mesurer les quantités de liquide de dialyse sont des débitmètres volumétriques, il est indispensable de dégazer le liquide de dialyse pour obtenir une mesure précise.

Selon une première variante de réalisation du rein artificiel objet de l'invention représenté sur le schéma de la figure IV, le liquide de dialyse est extrait du circuit (5) au niveau du dispositif de dégazage, au moyen de la pompe (17). Le niveau du liquide de dialyse dans le réservoir (20) est contrôlé par des électrodes. La mise en oeuvre de la pompe d'extraction (17) permet à la fois de maintenir le niveau du liquide de dialyse constant dans le réservoir (20) et d'extraire une fraction du liquide de dialyse. La quantité de liquide de dialyse extrait que l'on considère égale à la quantité d'ultrafiltrat retiré du sang est mesurée par tous moyens connus, par exemple par une éprouvette. Des essais effectués dans les conditions de réalisation de l'invention selon ce schéma ont donné les résultats suivants.

Sur dix essais réalisés en faisant circuler le liquide de dialyse à 500 ml/mn pendant 4 heures, la quantité totale de liquide de dialyse circulant dans le circuit (5), soit 120 l, a pu être mesurée à plus ou moins 20 ml près, soit une erreur moyenne de plus ou moins 5 ml/heure.

Ces résultats permettent de satisfaire aux exigences actuelles de précision concernant le contrôle de l'ultrafiltration.

Une seconde variante de réalisation du rein artificiel selon l'invention est représentée sur le schéma de la figure V. Dans ce cas, l'ultrafiltration n'est pas imposée par une dépression créée dans le compartiment (2) de l'hémodialyseur-par une extraction hors du circuit (5) d'une quantité de liquide de dialyse, mais en augmentant, par tous moyens connus, la pression du sang dans le compartiment (1) de l'hémodialyseur.

Les quantités de liquide de dialyse circulant dans le capteur amont (6) étant maintenues égales aux quantités de liquide de dialyse circulant dans le capteur aval (7) pendant la même période , l'excès de liquide de dialyse provenant de l'ultrafiltration du sang peut s'écouler par débordement au niveau du réservoir (20) de dégazage. On mesure la quantité de liquide de dialyse qui a débordé que l'on considère égale à la quantité d'ultrafiltrat provenant du sang.

Selon une autre variante de réalisation représentée sur la figure VI , on dispose en amont de l'hémodialyseur, non pas une pompe (9) mais une résistance hydraulique (22) constituée par exemple par un clamp variable ou une vanne pilotée. Le circuit de dérivation (13) relie la portion amont du circuit de liquide de dialyse comprise entre le capteur amont (6) et la résistance hydraulique (22) à la portion aval dudit circuit de liquide de dialyse comprise entre l'hémodialyseur (3) et la pompe (10). Dans ce mode de réalisation de l'objet de l'invention, il est souhaitable de disposer en amont du capteur amont (6) d'un dispositif, par exemple une pompe ou un réservoir en charge, (non représenté) permettant de fournir, au besoin, une pression suffisante au liquide de dialyse circulant dans le capteur amont (6).

Une vanne (11) est disposée sur le circuit de liquide de dialyse, en aval du point (B) et en amont de la résistance hydraulique (22). Une autre vanne (21) est disposée en aval de l'hémodialyseur (3) et en amont du point (E). Une troisième vanne (12) est disposée sur le circuit de dérivation (13).

Lors de l'étalonnage, on ouvre la vanne (12) et on ferme les vannes (11,21). Les quantités de liquide de dialyse circulant dans les capteurs amont et aval (6,7) sont fixées par le fonctionnement de la pompe (10). Ensuite, on ferme la vanne (12) et on ouvre les vannes (11,21). Le dispositif de contrôle (8) asservit la résistance hydraulique (22) pour maintenir les quantités de liquide de dialyse circulant dans le capteur amont (6) égales aux quantités de liquide de dialyse circulant dans le capteur aval (7).

Selon une autre variante de réalisation de l'objet de l'invention représentée sur le schéma de la figure VII, le liquide de dialyse extrait du circuit (5) par la pompe (17) est réinjecté dans le circuit de liquide de dialyse en amont du capteur amont (6).

Parmi les capteurs convenant pour la mise en oeuvre de la présente invention on peut citer divers débitmètres utilisables sur le circuit de liquide de dialyse, notamment les débitmètres électromagnétiques, thermiques, à ultra-sons, à engrenages, à effet Coriolis.

## Revendications

1. Procédé de contrôle et de maintien de l'égalité des quantités de liquide de dialyse à l'entrée et à la sortie d'un circuit de liquide de dialyse relié à un hémodialyseur (3) et comportant de part et d'autre dudit hémodialyseur au moins un capteur (6,7) apte à mesurer la quantité de liquide de dialyse circulant ainsi qu'un moyen (9,10) pour déplacer le liquide de dialyse, le procédé consistant à :
- mesurer la quantité de liquide de dialyse circulant dans le capteur (6) en amont de l'hémodialyseur,
- mesurer la quantité de liquide de dialyse circulant dans le capteur (7) en aval de l'hémodialyseur pendant la même période,
- comparer les quantités de liquide de dialyse circulant dans le capteur amont (6) et les quantités de liquide de dialyse circulant dans le capteur aval (7) pendant ladite même période, et
- asservir le fonctionnement de l'un des moyens pour déplacer le liquide de dialyse (9,10,22) au produit de la comparaison entre les quantités de liquide de dialyse circulant dans le capteur amont et les quantités de liquide de dialyse circulant dans le capteur aval, pendant la même période.

2. Procédé selon la revendication 1 caractérisé en ce qu'il consiste à extraire du circuit de liquide de dialyse une quantité de liquide de dialyse égale à la quantité d'ultrafiltrat que l'on veut retirer du sang.

3. Procédé selon la revendication 1 ou 2 caractérisé en ce qu'il consiste à étalonner les capteurs (6,7).

4. Rein artificiel comportant un hémodialyseur (3) comprenant au moins deux compartiments (1,2) séparés par une membrane semi-perméable permettant la dialyse et l'ultrafiltration du sang, le premier compartiment (1) étant destiné à être relié à un patient par un circuit extracorporel de sang (4), le second compartiment (2) étant traversé par un circuit de liquide de dialyse (5), ledit circuit de liquide de dialyse comportant en amont et en aval de l'hémodialyseur un moyen (9,10,22) pour déplacer le liquide de dialyse ainsi qu'un capteur (6,7) apte à mesurer la quantité de liquide de dialyse circulant et comportant en outre des moyens (35) pour comparer les valeurs obtenues par le capteur amont (6) et les valeurs obtenues par le capteur aval (7) pendant la même période, caractérisé en ce qu'il comporte des moyens pour maintenir l'égalité entre les valeurs obtenues par le capteur amont (6) et les valeurs obtenues par le capteur aval (7) pendant la même période, lesdits moyens comportant des moyens (36) pour asservir le fonctionnement de l'un au moins des moyens (9,10,22) pour déplacer le liquide de dialyse au produit de la comparaison entre les valeurs obtenues par le capteur amont (6) et les valeurs obtenues par le capteur aval (7) pendant la même période.

5. Rein artificiel selon la revendication 4, caractérisé en ce qu'il comporte des moyens (20,17) pour retirer hors du circuit (5) de liquide de dialyse une fraction du liquide de dialyse.

6. Rein artificiel selon la revendication 5, caractérisé en ce que lesdits moyens pour retirer une fraction du liquide de dialyse comportent notamment une pompe (17) pour imposer l'extraction d'une fraction de liquide de dialyse.

7. Rein artificiel selon l'une quelconque des revendications 4 à 6, caractérisé en ce que ledit moyen pour déplacer le liquide de dialyse (9,10) est une pompe occlusive.

8. Rein artificiel selon l'une quelconque des revendications 4 à 7, caractérisé en ce que lesdits capteurs pour mesurer les quantités de liquide de dialyse circulant comportent notamment des moyens pour générer des impulsions, chaque impulsion correspondant au passage d'une quantité prédéterminée de liquide de dialyse.

9. Rein artificiel selon l'une quelconque des revendications 4 à 8, caractérisé en ce que lesdits moyens pour comparer la valeur obtenue par le capteur amont (6) et la valeur obtenue par le capteur aval (7) comportent des moyens (30, 31) pour compter les impulsions émises par chacun desdits capteurs ainsi que des moyens (35) pour comparer le nombre d'impulsions émises par le capteur amont (6) et le nombre d'impulsions émises par le capteur aval (7) pendant la même période.

10. Rein artificiel selon l'une quelconque des revendications 4 à 9, caractérisé en ce qu'il comporte des moyens pour effectuer un étalonnage desdits capteurs.

11. Rein artificiel selon la revendication 10, caractérisé en ce que lesdits moyens pour effectuer un étalonnage desdits capteurs comportent des moyens (13) pour court-circuiter l'hémodialyseur et le circuit d'ultrafiltration, des moyens (32) pour déterminer un facteur de correction, ainsi que des moyens (33) pour mémoriser ledit facteur de correction.

12. Rein artificiel selon l'une quelconque des revendications 4 à 11, caractérisé en ce qu'il comporte en outre des moyens (20) pour éliminer les bulles de gaz présentes dans le liquide de dialyse.

## Claims

1. Method for controlling and maintaining the equality of dialysis liquid quantities at the inlet and outlet of a dialysis liquid circuit connected to a haemodialyser (3) and including on either side of the said haemodialyser, at least one sensor (6, 7) capable of measuring the circulating quantity of dialysis liquid, as well as a means (9, 10) for displacing the dialysis liquid, the method consisting in :
- measuring the quantity of dialysis liquid circulating in the sensor (6) upstream from the haemodialyser,
- measuring the quantity of dialysis liquid circulating in the sensor (7) downstream from the haemodialyser during the same period,
- comparing the quantities of dialysis liquid circulating in the upstream sensor (6) and the quantities of dialysis liquid circulating in the downstream sensor (7) during the said same period, and
- making the operation of one of the means (9, 10, 22) for displacing the dialysis liquid dependent on the result of the comparison between the quantities of dialysis liquid circulating in the upstream sensor and the quantities of dialysis liquid circulating in the downstream sensor during the same period.

2. Method according to claim 1, characterized in that it consists in extracting from the dialysis liquid circuit a quantity of dialysis liquid equal to the quantity of ultrafiltrate which one wishes to remove from the blood.

3. Method according to claim 1 or 2, characterized in that it consists in calibrating the sensors (6, 7).

4. An artificial kidney including a haemodialyser (3) comprising at least two compartments (1, 2) separated by a semi permeable membrane permitting the dialysis and ultrafiltration of the blood, the first compartment (1) being intended to be connected to a patient by an extracorporeal blood circuit (4), the second compartment (2) being traversed by a dialysis liquid circuit (5), the said dialysis liquid circuit including upstream and downstream from the haemodialyser, a means (9, 10, 22) for displacing the dialysis liquid, as well as a sensor (6, 7) capable of measuring the quantity of the circulating dialysis liquid and including, moreover, means (35) for comparing the values obtained by the upstream sensor (6) and the values obtained by the downstream sensor (7) during the same period, characterized in that it includes means for maintaining the equality between the values obtained by the upstream sensor (6) and the values obtained by the downstream sensor (7) during the same period, the said means including means (36) for making the operation of at least one of the means (9, 10, 22) for displacing the dialysis liquid dependent on the result of the comparison between the values obtained by the upstream sensor (6) and the values obtained by the downstream sensor (7) during the same period.

5. An artificial kidney according to claim 4, characterized in that it includes means (20, 17) for withdrawing from the dialysis liquid circuit (5) a fraction of the dialysis liquid.

6. An artificial kidney according to claim 5, characterized in that the means for withdrawing a fraction of the dialysis liquid include in particular a pump (17) for obtaining the extraction of a fraction of the dialysis liquid.

7. An artificial kidney according to any one of claims 4 to 6, characterized in that the said means (9, 10) for displacing the dialysis liquid is an occlusive pump.

8. An artificial kidney according to any one of claims 4 to 7, characterized in that the said sensors for measuring the quantities of circulating dialysis liquid include in particular means for generating pulses, each pulse corresponding to the passing of a predetermined quantity of dialysis liquid.

9. An artificial kidney according to any one of claims 4 to 6, characterized in that the said means for comparing the value obtained by the upstream sensor (6) and the value obtained by the downstream sensor (7) include means (30, 31) for counting the pulses emitted by each one of the said sensors, as well as means (35) for comparing the number of pulses emitted by the upstream sensor (6) and the number of pulses emitted by the downstream sensor (7) during the same period.

10. An artificial kidney according to any one of claims 4 to 9, characterized in that it includes means for effecting a calibration of the said sensors.

11. An artificial kidney according to claim 10, characterized in that the said means for effecting a calibration of the said sensors include means (13) for bypassing the haemodialyser and the ultrafiltration circuit, means (32) for determining a correction factor, as well as means (33) for storing the said correction factor.

12. An artificial kidney according to any one of claims 4 to 11, characterized in that it includes, moreover, means (20) for eliminating gas bubbles present in the dialysis liquid.

## Patentansprüche

1. Steuerungs- und Erhaltungsverfahren, um die Dialyseflüssigkeitsmengen am Eingang und am Ausgang eines Dialyseflüssigkeitskreislaufes, der mit einem Hämodialysator (3) verbunden ist, gleichzuhalten, und mit zumindest einem Messfühler (6, 7) zu beiden Seiten des Hämodialysators, der geeignet ist, die zirkulierende Dialyseflüssigkeitsmenge zu messen, sowie mit einem Mittel (9, 10), um die Dialyseflüssigkeit zu verschieben, wobei das Verfahren daraus besteht:
- die im oberstromigen Messfühler des Hämodialysators zirkulierende Dialyseflüssigkeitsmenge zu messen,
- die im unterstromigen Messfühler des Hämodialysators zirkulierende Dialyseflüssigkeitsmengen im gleichen Zeitabschnitt zu messen,
- die im oberstromigen Messfühler zirkulierende Dialyseflüssigkeitsmengen und die im unterstromigen zirkulierende Flüssigkeitsmengen im gleichen Zeitabschnitt zu vergleichen, und
- die Funktionsweise von zumindest einem der die Dialyseflüssigkeit verschiebenden Mittel zu steuern, gemäss dem Ergebnis aus dem Vergleich zwischen den im oberstromigen Messfühler (6) zirkulierende Dialyseflüssigkeitsmengen und den im unterstromigen Messfühler (7) zirkulierenden Dialyseflüssigkeitsmengen (9,10, 22) im gleichen Zeitabschnitt.

2. Verfahren nach Anspruch 1, dadurch gekennzeichnet, dass es daraus besteht, aus dem Dialyseflüssigkeitskreislauf eine Menge an Dialyseflüssigkeit gleich der Ultrafiltratsmenge, die man aus dem Blut ziehen will, herauszuziehen.

3. Verfahren nach Anspruch 1 oder 2, dadurch gekennzeichnet, dass es daraus besteht, die Messfühler (6,7) zu eichen.

4. Künstliche Niere, die einen Hämodialysator (3) aufweist, der wenigstens zwei Abteilungen (1, 2) aufweist, die durch eine halbdurchlässige Membrane getrennt sind, die die Dialyse und Ultrafiltration des Blutes ermöglicht, wobei die erste Abteilung (1) dazu bestimmt ist, durch einen ausserkörperlichen Blutkreislauf (4) an einen Patienten angeschlossen zu sein, und die zweite Abteilung (2) von einem Dialyseflüssigkeitskreislauf (5) durchflossen wird, und der Dialyseflüssigkeitskreislauf stromauf und stromab des Hämodialysators ein Mittel (9, 10, 22) zur Verschiebung der Dialyseflüssigkeit, sowie einen Messfühler (6, 7), der zur Messung der Zirkulierenden Dialyseflüssigkeitsmenge geeignet ist, aufweist, und der ausserdem Mittel (35) zum Vergleich der vom oberstromigen Messfühler (6) erhaltenen Werte mit den vom unterstromigen Messfühler (7) erhaltenen Werte aufweist, dadurch gekennzeichnet, dass sie Mittel zur Erhaltung der Gleichheit zwischen den vom oberstromigen Messfühler (6) erhaltenen Werten und den vom unterstromigen Messfühler (7) im gleichen Zeitabschnitt erhaltenen Werte aufweist, wobei die genannten Mittel Mittel (36) aufweisen, die die Funktionsweise von wenigstens einem der die Dialyseflüssigkeit verschiebenden Mittel gemäss dem Ergebnis des Vergleichs zwischen den vom oberstromigen Messfühler (6) erhaltenen Werten und den vom unterstromigen Messfühler (7) im gleichen Zeitabschnitt erhaltenen Werten steuern.

5. Künstliche Niere nach Anspruch 4, dadurch gekennzeichnet, dass sie Mittel (20, 17) zum Herausziehen eines Bruchteils an Dialyseflüssigkeit aus dem Dialyseflüssigkeitskreislauf aufweist.

6. Künstliche Niere nach Anspruch 5, dadurch gekennzeichnet, dass die Mittel zum Herausziehen eines Bruchteils an Dialyseflüssigkeit insbesondere eine Pumpe (17) zur Regelung der Extraktion eines Bruchteils an Dialyseflüssigkeit aufweisen.

7. Künstliche Niere nach irgendeinem der Ansprüche 4 bis 6, dadurch gekennzeichnet, dass das Mittel zur Verschiebung der Dialyseflüssigkeit (9,10) eine occlusive Pumpe ist.

8. Künstliche Niere nach irgendeinem der Ansprüche 4 bis 7, dadurch gekennzeichnet, dass die Messfühler zur Messung der zirkulierenden Dialyseflüssigkeitsmengen insbesondere Mittel zur Regelung der Impulse aufweisen, wobei jeder Impuls dem Durchfluss einer vorbestimmten Dialyseflüssigkeitsmenge entspricht.

9. Künstliche Niere nach irgendeinem der Ansprüche 4 bis 8, dadurch gekennzeichnet, dass die Mittel zum Vergleich des vom oberstromigen Messfühler (6) erhaltenen Wertes und dem vom unterstromigen Messfühler (7) erhaltenen Wertes Mittel (30, 31) aufweisen, um die von jedem der Messfühler ausgesandten Impulse zu zählen, sowie Mittel (35) um die Anzahl der vom oberstromigen Messfühler (6) ausgesandten Impulse mit der Anzahl der vom unterstromigen Messfühler (7) im gleichen Zeitabschnitt ausgesandten Impulse zu vergleichen.

10. Künstliche Niere nach irgendeinem der Ansprüche 4 bis 9, dadurch gekennziechnet, dass sie Mittel zur Eichung der Messfühler aufweist.

11. Künstliche Niere nach Anspruch 10, dadurch gekennzeichnet, dass die Mittel zur Eichung der Messfühler Mittel (13) aufweisen, um den Hämodialysator und den Ultrafiltrationskreislauf Kurzzuschliessen, Mittel (32), um einen Korrekturfaktor festzulegen, sowie Mittel (33), um den Korrekturfaktor zu speichern.

12. Künstliche Niere nach irgendeinem der Ansprüche 4 bis 11, dadurch gekennzeichnet, dass sie ausserdem Mittel (20) aufweist, um die Gasblasen, die in der Dialyseflüssigkeit enthalten sind, zu entfernen.
